# EUROPÄISCHE PATENTSCHRIFT

(11) **EP 2 130 566 B1**
(45) Veröffentlichungstag und Bekanntmachung des Hinweises auf die Patenterteilung: **08.04.2015**
(21) Anmeldenummer: 09159864.9
(22) Anmeldetag: 11.05.2009
(51) Int. Cl.: A61N 1/36, A61N 1/375, A61N 1/378, A61N 1/372

(54) **Langgestrecktes Implantat mit externer Energieeinkopplung**
Elongated implant with external energy coupling
Implant allongé doté d'un couplage d'énergie externe

(30) Priorität: 05.06.2008 DE 102008002228
(43) Veröffentlichungstag der Anmeldung: 09.12.2009
(73) Patentinhaber: Biotronik CRM Patent AG, 6341 Baar (CH)
(72) Erfinder: Weiss, Ingo, 12435, Berlin (DE); Dörr, Thomas, 12437, Berlin (DE); Neumann, Andreas, 12437, Berlin (DE); Flach, Erhard, 12305, Berlin (DE); Burch, André, 8184, Bachenbülach (CH)
(74) Vertreter: Lindner-Vogt, Karin L.

(56) Entgegenhaltungen:
- WO-A-2007/149462
- WO-A-2008/034005
- US-A- 5 193 540
- US-A1- 2002 095 191
- US-A1- 2004 088 024
- US-A1- 2006 085 041
- US-A1- 2008 021 505

## Beschreibung

Die Erfindung betrifft ein elektrotherapeutisches Implantat zur Stimulation von Körpergewebe.

Einsatzgebiete derartiger Instrumente sind insbesondere die Rückenmarksstimulation, die subkutane Stimulation (Elektroakupunktur), die Muskelstimulation, die kardiale sowie auch die Hirnstimulation. Kardial sind insbesondere rechtsventrikuläre Anwendungen in den Kammern und linksventrikuläre Anwendungen in den Gefäßen aber auch epikardiale Anwendungen bevorzugte Einsatzgebiete für das erfindungsgemäße Implantat.

Insbesondere betrifft die vorliegende Erfindung ein elektrotherapeutisches Implantat, dem die zur elektrotherapeutischen Behandlung von Körpergewebe notwendige Therapieenergie von außerhalb des Körpers zugeführt werden kann.

Prinzipiell sind derartige Implantate bekannt, weisen jedoch eine Reihe von Nachteilen auf. Die Größe und Bauform (Länge-Breite-Höhe-Relation) von elektrotherapeutischen Implantaten gemäß dem Stand der Technik ist im Wesentlichen durch die Größe und die Bauform der Batterie bestimmt.

Batterien besitzen allerdings eine Größe und eine Bauform, aufgrund derer sie oftmals nicht direkt am Therapie-Ort untergebracht werden können.

Aus diesem Grund sind elektrotherapeutische Implantate gemäß dem Stand der Technik oft zweiteilig ausgelegt und umfassen neben einem Gehäuse, welches die Batterie und andere elektronische Bauteile beherbergt, noch separate Elektroden, welche über elektrische Zuleitungen mit den elektronischen Bauteilen innerhalb des Gehäuses verbunden sind.

Das Gehäuse derartiger Implantate wird normalerweise an einem therapiefernen Ort wie z.B. unterhalb der Brustmuskulatur angebracht. Die gewünschte Stimulation erfolgt über die am Therapieort angebrachten und mit den elektronischen Komponenten des Gehäuses verbundenen Elektroden. Das Gehäuse sendet dabei während der Stimulation die notwendige Therapieenergie über die elektrischen Zuleitungen.

Die WO2008/034005 A2 beschreibt ein Gerät zur Stimulation des linken Ventrikels, wobei die Energieübertragung kabellos erfolgt, Ähnliches wird auch in der US2002/0095191 A1 offenbart, wobei dort ein Schrittmacher RF-Impulse in einen Stimulationsimpuls umwandelt. Auch in der US2006/0085041A1 wird ein kabelloser Herzschrittmacher beschrieben. Die US2004/0088024 beschreibt eine Technologie zur Stimulation von Gehirngewebe.

Ein implantierbares Gerät gemäß dem Stand der Technik trägt aufgrund dessen Größe und dessen Bauform, insbesondere aufgrund der Größe und der Bauform der Batterie auf und ist an manchen Körperregionen besonders störend (z.B. wenn man darauf liegt).

Die Aufgabe der vorliegenden Erfindung besteht demnach darin, die sich aus dem Stand der Technik ergebenden Nachteile, insbesondere solche, die sich auf den Tragekomfort eines Implantats beziehen, zu beseitigen.

Erfindungsgemäß wird diese Aufgabe durch ein elektrotherapeutisches Implantat nach Anspruch 1 gelöst.

Das erfindungsgemäße Implantat ist gemäß eines weiteren Erfindungsgedankens Teil eines Systems, das zur Bereitstellung der einzukoppelnden Therapieenergie einen externen Impulsgenerator aufweist, der selbst auch einen weiteren Erfindungsgedanken darstellt.

Dieser externe Impulsgenerator ist dazu ausgebildet, im Behandlungsfall eine Therapieenergie mittels kontaktloser Energieübertragung in das Implantat einzukoppeln. Vorzugsweise verfügt der externe Impulsgenerator aus diesem Grund über wenigstens eine Antenne zur elektromagnetischen Übertragung einer hochfrequenten Wechselspannung.

Da die Therapieenergie bevorzugt als hochfrequente Wechselspannung in das Implantat eingekoppelt wird, verfügt das Implantat über eine Demodulationseinheit um die Abgabe eines demodulierten Impulses über die Elektrodenpole zu ermöglichen.

Gemäß einer besonders einfachen Ausführungsform ist die Demodulationseinheit eine Diode, welche auf der einen Seite mit der Zuleitung für die erste Elektrode und auf der anderen Seite mit der Zuleitung für die zweite Elektrode verbunden ist.

Die Elektroden des Implantats sind dabei bevorzugt mit einer elektrochemisch aktiven Beschichtung aus Iridium, Iridiumoxid, Carbon oder Titannitrid beschichtet.

Die Zuleitungen zu den Elektroden fungieren dabei als Antenne. Im Behandlungsfall wird eine über die Antenne eingekoppelte hochfrequente Wechselspannung durch die Diode gleichgerichtet, demoduliert und über die beiden Elektrodenpole abgegeben.

Besonders bevorzugt ist wenigstens die als Antenne ausgebildete Zuleitung des Implantats eine Wendel oder ein Seil aus Edelstahl, aus MP35N, aus DFT-Draht, aus leitfähigem Kunststoff, aus Carbonfasem, aus leitfähigen Flüssigkeiten oder leitfähigen Gelen.

Vorzugsweise besitzt das erfindungsgemäße Implantat eine Bauform, die so gestaltet ist, dass sie an die anatomischen Gegebenheiten des Zielgebietes angepasst ist.

Besonders bevorzugt besitzt das erfindungsgemäße Implantat eine langgestreckte Bauform, dessen Längsmaße zu den Quermaßen ein Verhältnis von besonders bevorzugt > 10 besitzen. Besonders bevorzugt ist das Implantat im Wesentlichen zylinderförmig und mit einem kreisförmigen Querschnitt ausgeführt.

Gemäß einer besonders bevorzugten Ausführungsform besitzt das Implantat einen Durchmesser von weniger als 3,3 mm.

Um dem Patienten einen möglichst angenehmen Tragekomfort sicherzustellen, ist das Implantat vorzugsweise mechanisch flexibel ausgebildet und kann sich dem Körper auch während der Bewegung anpassen.

Um auch nach längeren Tragzeiten im Körper eines Patienten gegen Funktionsstörungen abgesichert zu sein, sind alle Verbindungen des Implantats vorzugsweise ermüdungssicher ausgeführt.

Um trotzdem an dem gewünschten Therapieort fixiert werden zu können, besitzt das Implantat gemäß einer bevorzugten Ausführungsvariante Mittel zu dessen Fixierung am Therapieort. Besonders bevorzugt verfügt das Implantat sowohl über wenigstens eine Schlaufe oder Öse in einem proximalen Bereich des Implantats, über die es am Körpergewebe festgenäht werden kann als auch und über ein distal angeordnetes Ankerelement, wie z.B. Tines, eine Fixierschraube, eine Helix, einen Ballon, eine stentartige Vorrichtung oder auch ein Vorformung in einem distalen Bereich des Implantats, welche mit den Gefäßabzweigungen einen Formabschluss bilden kann.

Neben seiner langgestreckten, besonders bevorzugt zylindrisch ausgebildeten Form mit besonders bevorzugt isodiametrischem Querschnittsverlauf über die gesamte Länge, besitzt das Implantat gemäß einer weiteren Ausführungsvariante einen Abschnitt mit vergrößertem Durchmesser, in welchem einzelne, größere elektronische Komponenten untergebracht sind.

Der Abschnitt des vergrößerten Außendurchmessers ist so bemessen, dass das längsgestreckte Implantat dennoch in Blutgefäße eingeführt werden kann.

Gemäß einer weiteren Ausführungsvariante ist das Implantat nicht über den gesamten Verlauf isodiametrisch ausgebildet, sondern verjüngt sich im Querschnitt zu einem distalen Ende hin, welches aufgrund seines verjüngten Querschnittes besonders einfach in ein Blutgefäß eingeführt werden kann und besonders weit in die peripher dünner werdenden Blutgefäße vorgeschoben werden kann.

Um ein noch leichteres Verbringen des Implantats an den Behandlungsort zu ermöglichen, ist gemäß einer weiteren Ausgestaltung ein Mittel zur Steuerung des Implantats innerhalb des Körpers oder der Körperhöhlen vorgesehen. Gemäß einer Ausführungsvariante ist hierfür ein in Längsrichtung durchgehendes Lumen zur Aufnahme eines Führungsdrahtes vorgesehen. Zur Verbringung des Implantats an den Behandlungsort wird in einem solchen Fall zunächst ein Führungsdraht in ein Blutgefäß eingeführt und dann das erfindungsgemäße Implantat über den Führungsdraht vorgeschoben, bis es seine gewünschte Position erreicht hat.

Gemäß einer besonders bevorzugten Ausführungsvariante verfügt das Implantat über eine Freischaltungsvorrichtung die eine Abgabe der über die Antenne empfangenen Therapieenergie nur bei Empfang eines entsprechenden Freischaltungscodes zulässt.

Eine Überprüfung der Zulässigkeit einer Freischaltung dient dazu, dass ausschließlich eine zur Therapie vorgesehene Therapieenergie, welche über die Antenne des Implantats eingekoppelt worden ist, über die Elektrodenpole abgegeben werden kann.

Wird in das Implantat unbeabsichtigt eine Therapieenergie eingekoppelt, werden die Elektrodenpole nicht freigeschaltet, da kein notwendiger Freischaltungscode vorliegt.

Der zur Freischaltung der Elektrodenpole notwendige Code kann dabei gemäß einer Ausführungsvariante über die Antenne zur Einkoppelung der Therapieenergie empfangen werden. Gemäß einer alternativen, bevorzugten Ausführungsform ist eine zweite Antenne vorgesehen, welche für den Empfang von Steuersignalen und/oder Freischaltungscodes ausgelegt ist.

Gemäß einer besonders einfachen Ausführungsvariante wird die Freischaltungsvorrichtung von einem Feldeffekttransistor gebildet, der zunächst die Abgabe von Therapieenergie über die Elektrodenpole blockiert. Mit dem Feldeffekttransistor ist bei dieser Ausführungsform die zweite Antenne derart verbunden, dass die hierüber empfangenen Steuersignale und/oder Freischaltungscodes mit Hilfe der Sperrschicht des Feldeffekttransistors die Freischaltung der Elektrodenpole steuern.

Wird über die Antenne für den Empfang von Steuersignalen und/oder Freischaltungscodes eine ausreichende Leistung eingekoppelt, schaltet der Feldeffekttransistor die Elektrodenpole frei und die Therapieenergie wird im Behandlungsfall an das zu stimulierende Körpergewebe abgegeben.

Gemäß einer bevorzugten Ausführungsvariante ist der Feldeffekttransistor ein JFET-Transistor.

Eine gesonderte Ausbildung einer Antenne zum Empfang von Steuersignalen und/oder Freischaltungscodes ist deswegen von Vorteil, weil auf diese Art und Weise möglich ist, diese Antenne für eine andere Resonanzfrequenz auszulegen als die Antenne zur Einkoppelung der Therapieenergie. Dadurch ist es möglich, auf ein nachträgliches Herausfiltern von Steuersignalen und/oder Freischaltungscodes aus einem die Therapieenergie enthaltenden Signal zu verzichten. Gemäß einer alternativen Ausführungsvariante kann das Implantat jedoch auch zum Herausfiltern von Steuersignalen und/oder Freischaltungscodes aus einem die Therapieenergie enthaltenden Signal ausgebildet sein.

Anstatt einer zweiten Antenne kann das Implantat auch über Mittel Verfügen, um Steuersignale, die in Form eines modulierten Magnetfeldes oder durch Ultraschall von extern gesendet werden, empfangen zu können.

Um zu verhindern, dass ein Steuersignal und/oder ein Freischaltungscode, welcher für einen anderen Patienten bestimmt ist, fälschlicherweise in das Implantat eingekoppelt wird und fälschlicherweise bewirkt, dass eine in das Implantat eingekoppelte Therapieenergie über die Elektrodenpole abgegeben wird, ist das erfindungsgemäße Implantat gemäß einer bevorzugten Ausführungsvariante dazu ausgebildet, erst bei Empfang eines Freischaltungscodes, welcher eine implantatsindividuelle Kennung enthält, die Elektrodenpole freizuschalten.

Eine derartige Ausführungsvariante verfügt zu diesem Zweck über eine Steuereinheit, die die implantatsindividuelle Kennung eines empfangenen Freischaltungscodes überprüft und erst bei Empfang einer korrekten Kennung eine Freischaltung der Elektrodenpole zulässt.

Gemäß einer weiteren Ausführungsvariante ist die Steuereinheit, welche bevorzugt auch die Funktion der Demodulationseinheit und der Freischaltungsvorrichtung beinhaltet, dazu ausgebildet, bei Empfang eines entsprechenden Steuersignals eine Therapiesequenz (Ansteuerung der Elektrodenpole, Reihenfolge, Polarität, Pulsparameter, Impulsamplitude etc.) und die Therapiedauer zu regeln.

Verfügt das Implantat über eine Vielzahl von Elektroden, ist die Steuereinheit bevorzugt auch dazu ausgebildet, für die Abgabe eines bestimmten Impulses ausschließlich eine bestimmte Untergruppe von Elektrodenpolen freizuschalten.

Zum Empfang entsprechender Steuersignale ist die Steuereinheit vorzugsweise wiederum mit der Antenne zum Empfang von Steuersignalen und/oder Freischaltungscodes verbunden.

Zur Energieversorgung ist die Steuereinheit vorzugsweise mit der Antenne zur Einkoppelung der Therapieenergie verbunden und nutzt einen Teil der eingekoppelten Therapieenergie für ihre Energieversorgung.

Gemäß einer weiteren Ausführungsvariante ist die Steuereinheit so ausgebildet, dass die zur Versorgung der Steuereinheit notwendige Energie über die Antenne zur Übertragung der Steuersignale und/oder Freischaltungscodes eingekoppelt wird.

Gemäß einer weiteren Ausführungsvariante verfügt die Steuereinheit über einen temporären Energiespeicher, welcher in der Lage ist, eine von außen eingekoppelte Energie zur Versorgung der Steuereinheit zu speichern und kontinuierlich der Steuereinheit zur Verfügung zu stellen.

Der temporäre Energiespeicher zur Versorgung der Steuereinheit ist dabei gemäß einer Ausführungsvariante mit der Antenne zur Einkoppelung der Therapieenergie oder gemäß einer anderen Ausführungsvariante mit der Antenne zur Einkoppelung von Steuersignalen und/oder Freischaltungscodes verbunden.

Gemäß einer weiteren Ausführungsvariante besitzt das Implantat auch einen Anschluss zum Anschließen eines herkömmlichen Herzschrittmachers.

In Verbindung mit einem Herzschrittmacher werden die Elektrodenpole des Implantats als die mit einem herkömmlichen Herzschrittmacher zu verbindenden Elektrodenpole benutzt. Gemäß einer entsprechenden Ausführungsvariante ist das Implantat dazu ausgebildet, vom Herzschrittmacher erzeugte Impulse an die Elektrodenpole weiterzuleiten. Diese Ausführungsform besitzt den Vorteil, dass neben den vom Herzschrittmacher weitergeleiteten Signalen für bestimmte Behandlungsverfahren autark zum Herzschrittmacher Energie und Steuersignale direkt in das erfindungsgemäße Implantat eingekoppelt werden können, ohne dass der Energiespeicher des angeschlossenen Herzschrittmachers belastet wird.

Gemäß einer weiteren Ausführungsvariante verfügt die Steuereinheit des erfindungsgemäßen Implantats auch über eine Messvorrichtung, welche dazu ausgebildet ist, über Sensoren ermittelte Patientenbezogene Messdaten oder Gerätebezogene Messdaten über eine Antenne nach extern zu übertragen.

Vorzugsweise ist die Messvorrichtung dabei mit der Antenne für die Übertragung der Steuersignale und/oder Freischaltungscodes verbunden und sendet über diese die patienten- und/oder Gerätebezogenen Messdaten nach extern.

Der externe Impulsgenerator ist zur externen Versorgung des Implantats mit Therapieenergie mit einem Hochfrequenzgenerator und wenigstens einer Antenne ausgestattet, über die im Anwendungsfall Therapieenergie an das Implantat gesendet wird.

Vorzugsweise verfügt der Impulsgenerator zusätzlich über eine Steuereinheit, mit welcher Steuersignale generiert werden und über die Antenne zur Übertragung der Therapieenergie oder gemäß einer zweiten Ausführungsvariante über eine zweite Antenne des Impulsgenerators an das Implantat übertragen werden können.

Anstatt einer zweiten Antenne ist der Impulsgenerator gemäß einer weiteren Ausführungsform dazu ausgebildet, die Steuersignale im Behandlungsfall mittels eines von der Steuereinheit des Impulsgenerators modulierten Magnetfeldes oder per Ultraschall an das Implantat zu übertragen.

Bei den Steuersignalen, welche im Behandlungsfall an das Implantat übertragen werden, handelt es sich vorzugsweise um Freischaltungscodes, welche - wie weiter oben beschrieben - im Behandlungsfall zur Freischaltung des Implantats führen. Diese Freischaltungscodes enthalten vorzugsweise implantatsindividuelle Kennungen, sodass ausschließlich ein Implantat mit dem externen Impulsgenerator freigeschaltet werden kann, welches die gesendete implantatsindividuelle Kennung akzeptiert.

Gemäß einer weiteren Ausführungsform ist die Steuereinheit des Impulsgenerators dazu ausgebildet, Steuersignale zu generieren, welche eine Information über eine gewünschte Therapiesequenz (Ansteuerung der Elektroden, Polarität, Pulsparameter, Impulsamplitude etc.) beinhalten.

Entsprechend einer weiteren Ausführungsvariante ist der Impulsgenerator dazu ausgebildet, von dem Implantat gesendete Patientenbezogene Messdaten und/oder Gerätebezogene Messdaten empfangen zu können und bevorzugt mit Hilfe der Steuereinheit des Impulsgenerators analysieren und/oder abspeichern zu können.

Zur Eingabe Patientenbezogener Parameter, die z. B. für die Generierung des Freischaltungscodes oder zur Generierung eines Steuersignals zur Steuerung der Therapiesequenz notwendig sind, verfügt der erfindungsgemäße Impulsgenerator bevorzugt auch über eine Dateneingabeschnittstelle.

Weitere Aspekte des erfindungsgemäßen Implantats und verschiedene Ausführungsvarianten sind anhand der Zeichnungen näher erläutert. Es zeigen:
- Fig. 1: eine perspektivische Außendarstellung des erfindungsgemäßen Implantats;
- Fig. 2: die Anordnung der elektronischen Komponenten des Implantats gemäß einer ersten Ausführungsform;
- Fig. 3: die Anordnungen der elektronischen Komponenten des Implantats gemäß einer zweiten Ausführungsform mit einer Freischaltungsvorrichtung;
- Fig. 4: die Anordnungen der elektronischen Komponenten des Implantats gemäß der zweiten Ausführungsform, wobei die Freischaltungsvorrichtung als Feldeffekttransistor ausgeführt ist;
- Fig. 5: die Anordnungen der elektronischen Komponenten des Implantats gemäß einer dritten Ausführungsform mit Steuereinheit und drei Elektrodenpolen;
- Fig. 6: die Anordnungen der elektronischen Komponenten des Implantats gemäß einer vierten Ausführungsform mit Steuereinheit, Messvorrichtung und drei Elektrodenpolen;
- Fig. 7: die elektronischen Komponenten des Implantats gemäß einer fünften Ausführungsform mit Steuereinheit und Anschluss zum Anschließen eines Herzschrittmachers; und
- Fig. 8: ein im Körper eines Patienten zu einer Schlaufe zusammen gebogenes Implantat, welches entsprechend einer Spule zur Erzeugung einer Induktionsspannung fungiert.

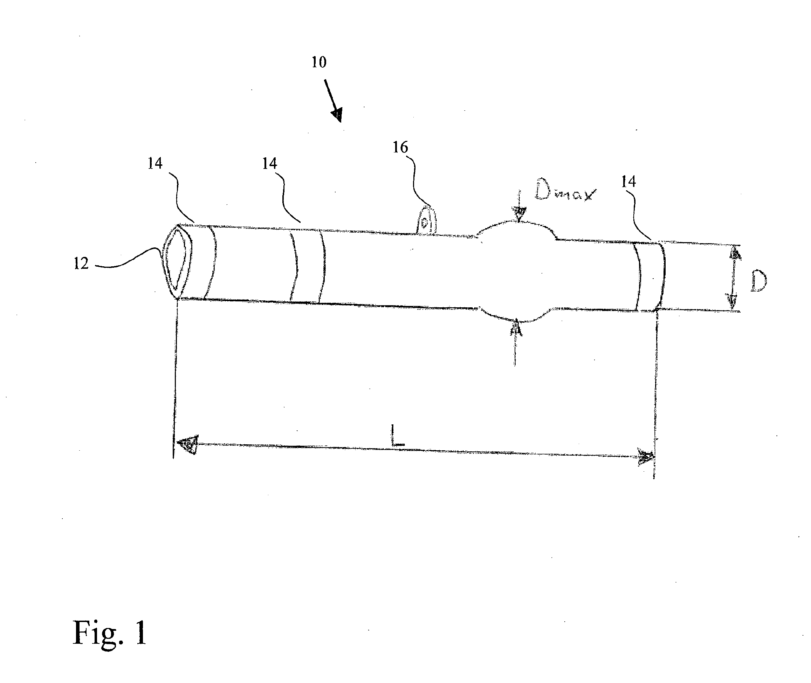

Fig. 1 zeigte eine perspektivische Außendarstellung des erfindungsgemäßen Implantats 10 mit der Länge L und dem Durchmesser D gemäß einer besonders bevorzugten Ausführungsvariante, bei der das Implantat als längsgestreckter Hohlzylinder ausgeführt ist, welcher ein in Längsrichtung durchgehendes Lumen aufweist und ein Streckungsverhältnis von L/D von >10 besitzt.

Die in Fig. 1 dargestellte Ausführungsvariante des Implantats 10 verfügt dabei über einen rohrförmigen Isolator 12, auf dem insgesamt drei ringförmige Elektroden 14 aufgezogen sind. Hier im Bild nicht erkennbar sind die elektronischen Komponenten des Implantats im Mantel des rohrförmigen Isolators 12 eingebettet. Gemäß der hier dargestellten Ausführungsvariante weitet sich der äußere Durchmesser des rohrförmigen Isolators 12 in einem bestimmten Abschnitt nach außen, welches den Zweck erfüllt, dass in diesem Bereich größere elektronische Komponenten, welche in dem Mantel des rohrförmigen Isolators 12 ansonsten nicht untergebracht werden können, Platz finden. Der Durchmesser Dₘₐₓ in diesem Bereich ist jedoch kleiner als der eineinhalbfache Durchmesser D des Isolators 12.

Zur Fixierung am Behandlungs-Ort verfügt das Implantat 10 über eine Schlaufe 16, über die es am Körpergewebe eines Patienten festgenäht werden kann.

Das in Fig. 1 dargestellte Implantat 10 mit einem durchgehenden Lumen besitzt den Vorteil, dass es über einen Führungsdraht besonders leicht und Risikominimierend an den gewünschten Therapieort verbracht werden kann.

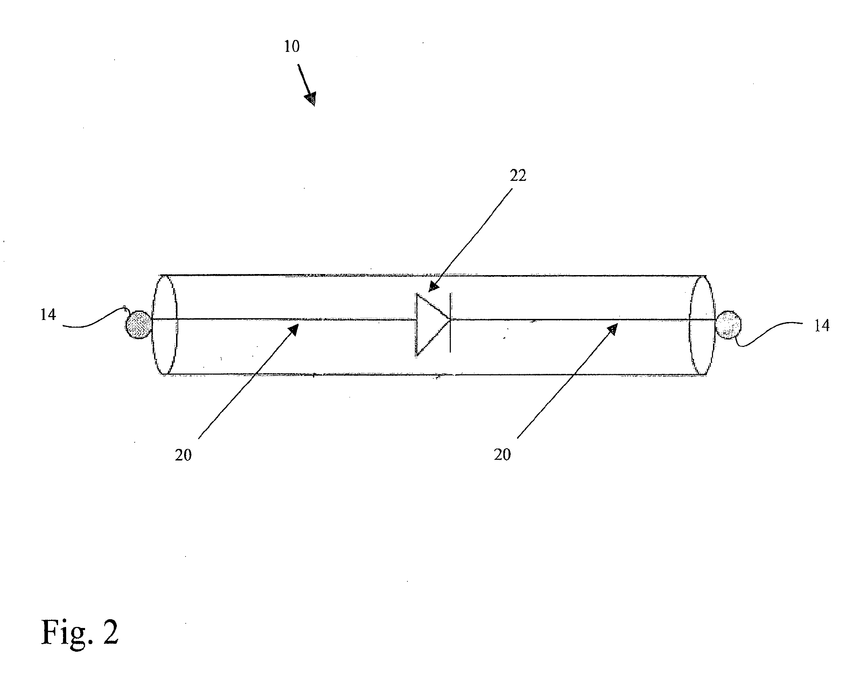

Fig. 2 zeigt die im Inneren eines zylinderförmig ausgeführten isolierenden Implantatgehäuses untergebrachten elektronischen Komponenten gemäß einer besonders einfachen Anordnungsvariante.

Der Fokus in den Figuren 2 bis 7 ist insbesondere auf die elektronischen Komponenten des Implantats gerichtet doch es sei an dieser Stelle angemerkt, dass die elektronischen Komponenten sowohl im Mantel eines rohrförmigen Isolators als auch in einem langgestreckten isolierenden Gehäuse ohne durchgehendem Lumen untergebracht sein können.

Ebenso sind die in den Figuren 2 bis 7 dargestellten Elektroden als Kugelelektroden ausgeführt. Dies ist jedoch nicht als beschränkend für die Erfindung aufzufassen und es kann bei jeder Ausführungsvariante grundsätzlich jede Art von Elektrode verwendet werden.

Gemäß der in Figur 2 dargestellten Ausführungsvariante sind an den beiden Längsenden des Implantats zwei Kugelelektroden 14 angeordnet, welche mit jeweils einer elektrischen Zuleitung 20 verbunden sind. Die Zuleitungen sind ihrerseits mit den beiden Anschlüssen einer Diode 22 verbunden.

Die Zuleitungen 20 fungieren dabei als Antenne, welche so ausgeführt ist, dass die Frequenz der einzukoppelnden Therapieenergie vorzugsweise der Resonanzfrequenz entspricht. Durch die Diode 22 wird dabei die eingekoppelte Hochfrequenz gleichgerichtet, demoduliert und führt an den beiden Elektrodenpolen 14 zum Anliegen einer pulsförmigen Spannung.

Im Behandlungsfall wird das Implantat 10 so im Körper des Patienten positioniert, dass die Elektrodenpole 14 das zu behandelnde Körpergewebe kontaktieren.

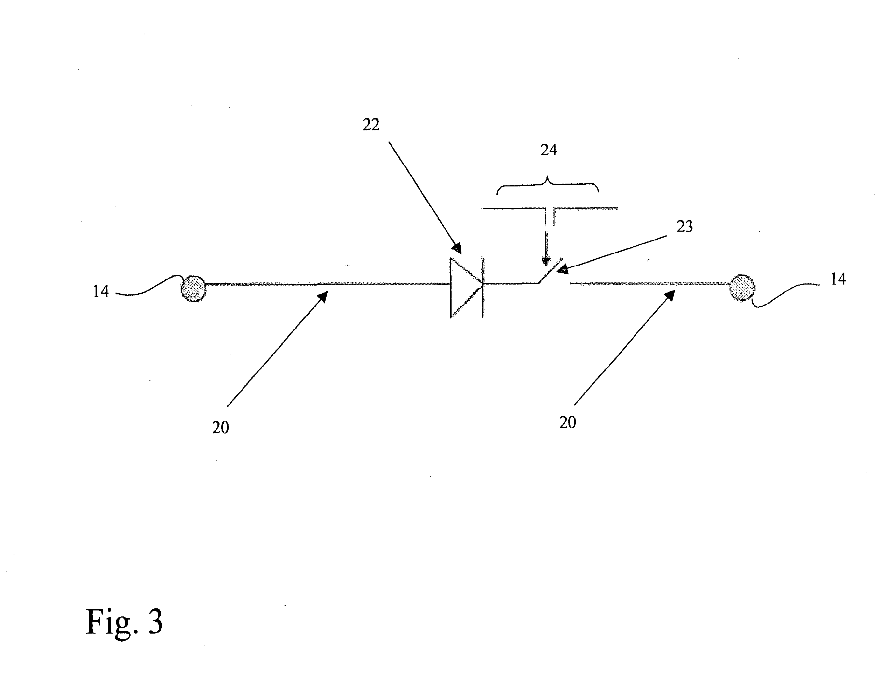

Fig. 3 zeigt eine zu Fig. 2 ähnliche Anordnung der elektronischen Komponenten des Implantats mit zwei Elektrodenpolen 14, die über die elektrischen Zuleitungen 20 mit den beiden Anschlüssen einer Diode 22 verbunden sind. Zwischen dem ausgangsseitigen Anschluss der Diode 22 und der entsprechenden elektrischen Zuleitung 20 ist gemäß dieser Ausführungsform eine Freischaltungsvorrichtung 23 angeordnet, welche dazu ausgebildet ist, eine im Betriebsfall anliegende Spannung für die Elektrodenpolen 14 entweder frei zu schalten oder zu sperren. Angedeutet ist hier bereits, dass zur Betätigung der Freischaltungsvorrichtung 23 eine weitere Antenne 24 vorgesehen ist.

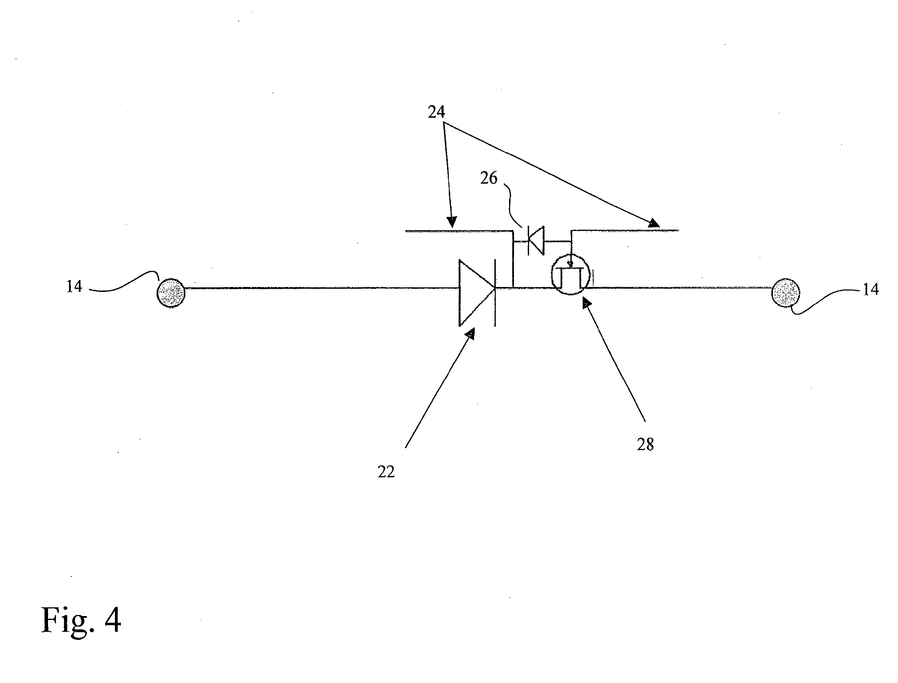

Fig. 4 zeigt die Anordnung der elektronischen Komponenten gemäß der Ausführungsform aus Fig. 3, wobei hier detailliert dargestellt wird, wie mit Hilfe der Antenne 24, welche zum Empfang von Steuersignalen und/oder Freischaltungscodes ausgebildet ist, ein Freischalten der Elektrodenpole 14 erfolgt.

Gemäß der hier dargestellten Ausführungsvariante dient als Freischaltungsvorrichtung 23 ein JFET-Transistor 28.

Die Freischaltvorrichtung 23 kann auch durch einen Reed-Kontakt realisiert werden. Die Steuerung erfolgt dann nicht über eine zusätzliche Antenne sondern durch ein von extern wirkendes Magnetfeld, das von der externen Steuereinheit moduliert wird. Statt eines Reed-Kontaktes können auch andere Magnetfeld-sensitive Bauelemente eingesetzt werden, z.B. Bauelemente, die einen Riesenmagnetwiderstand (GMR), anisotropen Magnetwiderstand (AMR), kolossalen Magnetwiderstandeffekt (CMR) oder magnetischen Tunnelwiderstand (TMR) besitzen. Ferner kann die Freischaltvorrichtung 23 auch über einen piezoelektrischen Wandler angesteuert werden. Das Steuersignal wird über mechanische Schwingungen eingekoppelt. Dies hat den Vorteil, dass diese Lösung über Elektromagnetische Einflüsse nicht störbar ist.

Zur Freischaltung der Elektrodenpole 14 wird in einem Ausführungsbeispiel mit Hilfe hochfrequenter Steuersignale und/oder Freischaltungscodes über die Antenne 24, welche ebenfalls über eine Diode 26 zur Gleichrichtung verfügt, eine gleichgerichtete, pulsförmige Spannung erzeugt, mit welcher die Durchleitung des JFET zwischen Diode 22 und dem Elektrodenpol 14 gesteuert wird. Die Antenne 24 ist dabei auf eine andere Frequenz abgestimmt als die Antenne 20. Auf diese Weise wird verhindert, dass fälschlicherweise in die Antenne 20 eingekoppelte Therapieenergie an die Elektrodenpole 14 weitergeleitet wird und eine unerwünschte Stimulation des zu behandelnden Gewebes erfolgt.

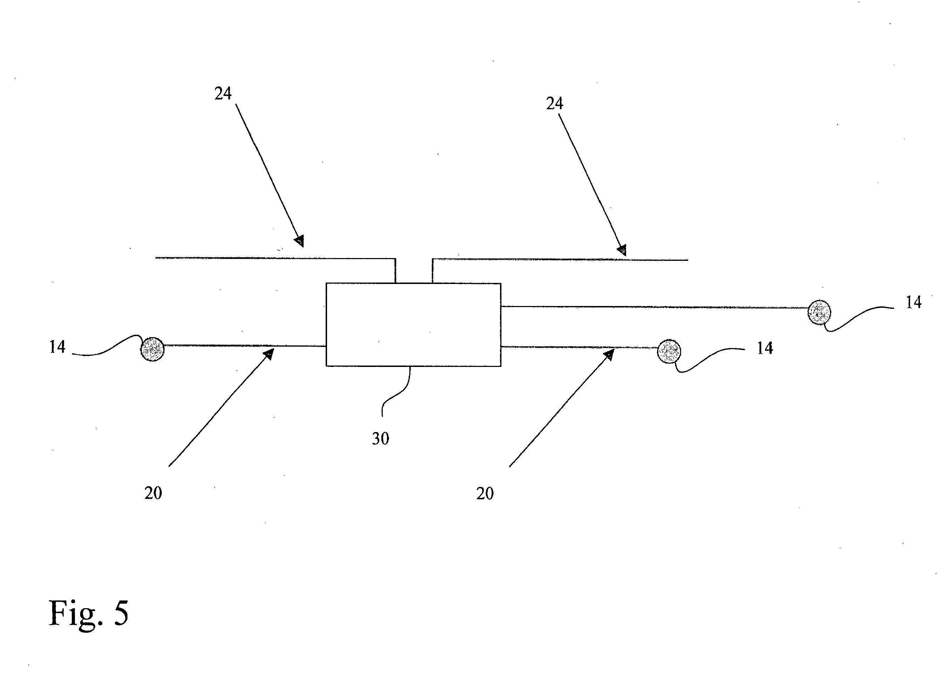

Fig. 5 zeigt eine weitere Ausführungsvariante des Implantats 10 mit drei Elektrodenpolen 14. Anstelle der Diode 22 und des JFET-Transistors 28 gemäß Fig. 4 verfügt die Variante gemäß Fig. 5 über eine Steuereinheit 30, welche dazu ausgebildet ist, die Gleichrichtung und die Demodulation einer eingekoppelten hochfrequenten Wechselspannung zu übernehmen. Gleichzeitig übernimmt die Steuereinheit 30 auch das Freischalten der drei Elektroden 14 in Abhängigkeit der über die Antenne 24 übertragenen Steuersignale und/oder Freischaltungscodes.

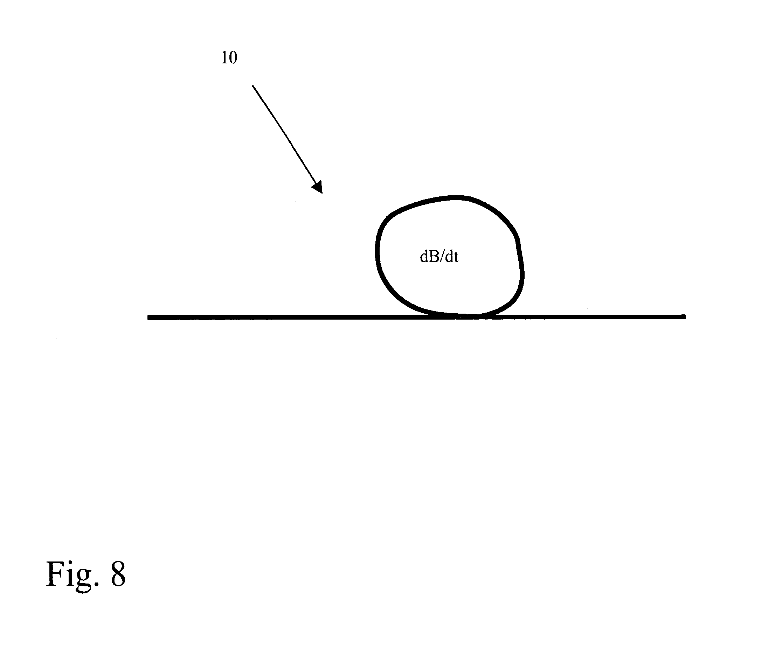

Die einzelnen Komponenten der Steuereinheit 30 sind hier nicht dargestellt, bewirken aber, dass eine Freischaltung der Elektrodenpole 14 so lange nicht erfolgt, bis ein Freischaltungscode, welcher eine korrekte implantatsindividuelle Kennung enthält, über die Antenne 24 empfangen wird. Die Rolle der Antenne 24 kann auch über eines der oben genannten Prinzipien (magnetisch oder per Ultraschall) realisiert sein. Ferner kann das Steuersignal auch über die Antenne 20 selbst ohne Notwendigkeit der Antenne 24 eingebracht werden. Ein Lösungsweg ist, das in die Antenne 20 energieeinbringende Signal zu modulieren (z. B. Amplituden- Frequenz oder Phasenmodulation). Ein anderer Lösungsweg ist, das langgestreckte Implantat - wie in Figur 8 dargestellt - so zu implantieren, dass es eine Schlaufe bildet, in die über ein variables Magnetfeld von extern eine Spannung induziert wird (z. B. gepulst), die die Steuerinformation trägt. Die Amplitude dieses so induzierten Signals ist dabei so gering zu halten, dass sie selbst nicht stimulieren kann; die Steuerinformation wird dann in der Steuereinheit per Signalverarbeitung extrahiert.

Hier nicht dargestellt verfügt die Steuereinheit 30 auch über einen temporären Energiespeicher, welcher dazu ausgebildet ist, die Steuereinheit 30 kontinuierlich mit Energie zu versorgen. Dieser Energiespeicher ist so ausgebildet, dass er über die Antenne zur Einkoppelung der Therapieenergie 20 aufgeladen wird.

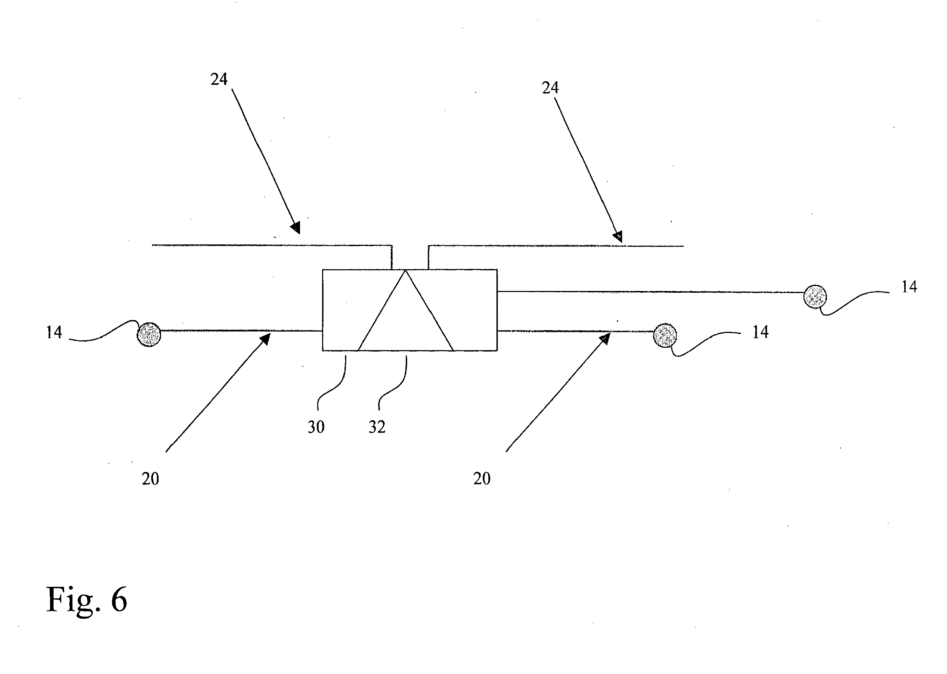

Die Ausführungsvariante des in Fig. 6 dargestellten Implantats 10 entspricht in weiten Teilen der Ausführungsvariante gemäß Fig. 5 mit dem Unterschied, dass die Steuereinheit 30 zusätzlich über eine Messvorrichtung 32 verfügt.

Die Messvorrichtung 32 ist dabei dazu ausgebildet, über die Elektrodenpole 14 empfangene Patientenbezogene elektrische Impulse (z. B. Nervensignale) zu messen und über die Antenne 24 nach extern zu versenden. Verschiedene hier nicht dargestellte Sensoren messen und/oder analysieren patienten- und/ Gerätebezogene Messdaten und senden diese über die Antenne 24 nach extern.

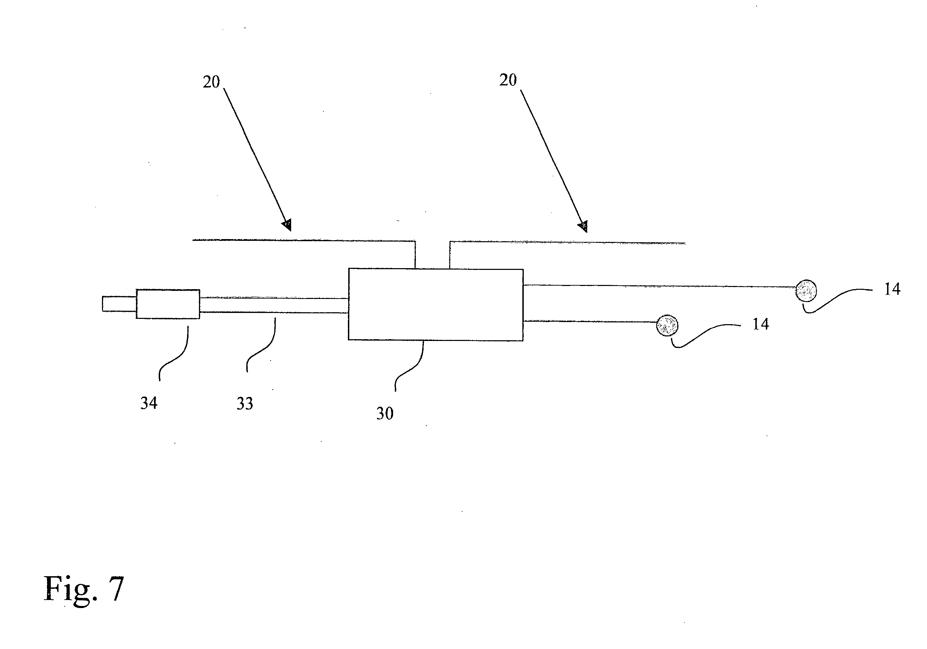

In Fig. 7 ist eine weitere Ausführungsvariante des erfindungsgemäßen Implantats 10 dargestellt, welche über eine Steuereinheit 30 verfügt, die über eine IPG-Steuerleitung 33 mit einem IS-1 Elektrodenstecker 34 verbunden ist, über welchen das Implantat auch mit einem herkömmlichen Herzschrittmacher verbunden werden kann.

## Patentansprüche

1. Elektrotherapeutisches Implantat (10) zur Stimulation von Körpergewebe mit
- wenigstens zwei Elektrodenpolen (14), die mit elektrischen Zuleitungen (20) verbunden sind,
- einer Demodulationseinheit (22),
- wenigstens einer elektrischen Zuleitung (20), die als Antenne ausgelegt ist und die Demodulationseinheit (22) kontaktiert,
wobei
- das Implantat (10) aus einem Stück mit geschlossener Außenform gefertigt ist, und
- über eine Steuereinheit (30) verfügt, die dazu ausgebildet ist, die Therapiesequenz zu steuern,
- Mittel (16) zu dessen Fixierung am Therapie-Ort besitzt und mit einer körperverträglichen Isolation ausgestattet ist, wobei
- die Komponenten des Implantats (10) so ausgebildet sind, dass eine während der Behandlung von extern über die Antenne (20) einkoppelbare Therapieenergie ohne Zwischenspeicherung über die Elektrodenpole (14) an das therapeutische Zielgebiet abgegeben wird,
**dadurch gekennzeichnet, dass** das Implantat:
- über eine Freischaltungsvorrichtung (23) verfügt, die dazu ausgebildet ist, erst bei Empfang eines Freischaltungscodes eine Abgabe von Therapieenergie über die Elektrodenpole (14) zuzulassen, und
- über eine zweite Antenne (24) verfügt, die dazu ausgebildet ist, einen zur Freischaltung der Elektrodenpole (14) notwendigen Freischaltungscode und/oder Steuersignale zu empfangen, wobei sich die Resonanzfrequenz der Antenne für das Freischaltungscode-Signal von der Resonanzfrequenz der Antenne für die empfangene Therapieenergie unterscheidet.

2. Implantat (10) nach Anspruch 1, mit einem Verhältnis von Längs- zu Quermaßen von ≥10.

3. Implantat (10) nach einem der vorangehenden Ansprüche, welches mit einem Mittel zu dessen Steuerung innerhalb des Körpers ausgestattet ist.

4. Implantat (10) nach einem der vorangehenden Ansprüche, **dadurch gekennzeichnet, dass** die empfangene Therapieenergie direkt an das therapeutische Zielgebiet abgegeben wird, indem die Antenne (20) an dem einen Ende die Demodulationseinheit (22) kontaktiert und am anderen Ende an einen weiteren Elektrodenpol (14) angeschlossen ist.

5. Implantat (10) nach einem der vorangehenden Ansprüche, **dadurch gekennzeichnet, dass** die Demodulationseinheit (22) eine Diode ist.

6. Implantat (10) nach Anspruch 1, **dadurch gekennzeichnet, dass** der zum Freischalten der Elektrodenpole notwendige Freischaltungscode eine implantatsindividuelle Kennung enthält.

7. Implantat (10) nach Anspruch 6, welches über einen temporären Energiespeicher verfügt, der die Steuereinheit (30) während der Behandlung mit Energie versorgt.

8. Implantat (10) nach einem der Ansprüche 6 oder 7, **dadurch gekennzeichnet, dass** die notwendige Energie zur Versorgung der Steuereinheit (30) über die Antenne (24) zur Übertragung der Steuersignale und/oder des Freischaltungscodes oder die Antenne zur Übertragung der Therapieenergie eingekoppelt wird.

9. Implantat (10) nach einem der vorangehenden Ansprüche, welches über wenigstens einen Anschluss (34) zum Anschließen eines herkömmlichen Herzschrittmachers verfügt.

10. Implantat (10) nach einem der vorangehenden Ansprüche, welches über eine Messvorrichtung (32) verfügt, die mit einer der Antennen (20, 24) verbunden ist und dazu ausgebildet ist, patienten- und/oder gerätebezogene Messdaten über die Antenne (20, 24) nach Extern zu übertragen.

11. Impulsgenerator zur externen Versorgung eines Implantats nach Anspruch 1 mit Therapieenergie mit
- einem Hochfrequenzgenerator zur Erzeugung einer hochfrequenten Wechselspannung, wenigstens
- einer Antenne, über die im Anwendungsfall Therapieenergie in Form einer hochfrequenten Wechselspannung gesendet wird und
- einer Steuereinheit, die dazu ausgebildet ist, ein Steuersignal zu generieren, das neben einem Freischaltungscode zur Freischaltung des Implantats eine Information über eine gewünschte Therapiesequenz enthält.

12. Impulsgenerator nach Anspruch 11, welcher dazu ausgebildet ist, von einem Implantat nach Anspruch 10 gesendete patientenbezogene Messdaten und/oder gerätebezogene Messdaten empfangen und analysieren und/oder abspeichern zu können.

## Claims

1. An electrotherapeutic implant (10) for stimulating bodily tissue, comprising
- at least two electrode poles (14), which are connected to electrical leads (20),
- a demodulation unit (22),
- at least one electrical lead (20), which is designed as an antenna and contacts the demodulation unit (22),
wherein
- the implant (10) is produced from a piece having a closed exterior shape, and
- comprises a control unit (30), which is designed to control the therapy sequence,
- comprises means (16) for the fixation thereof at the therapy site and is equipped with biocompatible insulation, wherein
- the components of the implant (10) are designed such that therapy energy that can be coupled in from the outside via the antenna (20) during treatment is delivered, without intermediate storage, to the therapeutic target area via the electrode pole (14),
**characterized in that** the implant:
- comprises a release device (23), which is designed to permit therapy energy to be delivered via the electrode pole (14) once a release code is received, and
- comprises a second antenna (24), which is designed to receive a release code, which is required in order to release the electrode pole (14), and/or control signals, wherein the resonant frequency of the antenna for the release code signal differs from the resonant frequency of the antenna for the therapy energy that is received.

2. The implant (10) according to claim 1, having a ratio of longitudinal dimension to transverse dimension of ≥10.

3. The implant (10) according to any one of the preceding claims, which is equipped with means for the control thereof within the body.

4. The implant (10) according to any one of the preceding claims, **characterized in that** the therapy energy that is received is delivered directly to the therapeutic target area **in that** the antenna (20) contacts the demodulation unit (22) at one end and, at the other end, is connected to another electrode pole (14).

5. The implant (10) according to any one of the preceding claims, **characterized in that** the demodulation unit (22) is a diode.

6. The implant (10) according to claim 1, **characterized in that** the release code that is required to release the electrode pole contains an implant-specific identifier.

7. The implant (10) according to claim 6, which has a temporary energy accumulator, which supplies the control unit (30) with energy during treatment.

8. The implant (10) according to any one of claims 6 or 7, **characterized in that** the energy required to supply the control unit (30) is coupled in via the antenna (24) for transmitting the control signals and/or the release code, or via the antenna for transmitting the therapy energy.

9. The implant (10) according to any one of the preceding claims, which has at least one connector (34) for the connection of a conventional cardiac pacemaker.

10. The implant (10) according to any one of the preceding claims, which has a measurement device (32), which is connected to one of the antennas (20, 24) and is designed to transmit patient- and/or device-specific measurement data via the antenna (20, 24) to the outside.

11. A pulse generator for the external supply of an implant according to claim 1 with therapy energy, comprising
- a high-frequency generator for generating a high-frequency alternating voltage, at least
- one antenna, via which therapy energy is transmitted in the form of high-frequency alternating voltage during use, and
- one control unit, which is designed to generate a control signal, which contains a release code for releasing the implant as well as information on a desired therapy sequence.

12. The pulse generator according to claim 11, which is designed to be capable of receiving and analyzing and/or storing patient-specific measurement data transmitted from an implant according to claim 10 and/or device-specific measurement data.

## Revendications

1. Implant électrothérapeutique (10) pour la stimulation de tissus corporels comprenant
- au moins deux pôles d'électrodes (14) qui sont reliés avec des lignes d'alimentation électriques (20),
- une unité de démodulation (22),
- au moins une ligne d'alimentation électrique (20) qui est conçue sous la forme d'une antenne et qui est en contact avec l'unité de démodulation (22),
où
- l'implant (10) est fabriqué en une seule pièce avec un contour extérieur fermé,
et
- dispose d'une unité de commande (30) qui est conçue pour conduire la séquence de thérapie,
- possède des moyens (16) pour sa fixation sur le site de la thérapie et est équipé d'une isolation compatible avec le corps, où
- les composants de l'implant (10) sont conçus de telle façon que, pendant le traitement, une énergie de thérapie, pouvant être induite par l'intermédiaire de l'antenne (20) sans stockage intermédiaire, est délivrée sur le site de la thérapie par l'intermédiaire des pôles d'électrodes (14), **caractérisé en ce que** l'implant :
- dispose d'un dispositif d'activation (23) qui est conçu pour ne permettre la fourniture de l'énergie de thérapie par l'intermédiaire des pôles d'électrodes (14) uniquement après la réception d'un code d'activation, et
- dispose d'une deuxième antenne (24) qui est conçue pour réceptionner le code d'activation nécessaire pour l'activation des pôles d'électrodes (14) et/ou des signaux de commande, la fréquence de résonance de l'antenne pour le signal du code d'activation étant différente de la fréquence de résonance de l'antenne pour l'énergie de thérapie réceptionnée.

2. Implant (10) selon la revendication 1, avec un rapport des dimensions longueur sur section ≥ 10.

3. Implant (10) selon l'une des revendications précédentes, lequel est équipé avec un moyen pour sa commande à l'intérieur du corps.

4. Implant (10) selon l'une des revendications précédentes, **caractérisé en ce que** l'énergie de thérapie réceptionnée est directement délivrée sur le site cible de la thérapie par le fait que l'antenne (20) est en contact à une extrémité avec l'unité de démodulation (22) et est raccordée à l'autre extrémité à un autre pôle d'électrode (14).

5. Implant (10) selon l'une des revendications précédentes, **caractérisé en ce que** l'unité de démodulation (22) est une diode.

6. Implant (10) selon la revendication 1, **caractérisé en ce que** le code d'activation nécessaire pour l'activation des pôles d'électrodes contient une identification individuelle de l'implant.

7. Implant (10) selon la revendication 6, qui dispose d'un accumulateur d'énergie temporaire qui alimente l'unité de commande (30) pendant le traitement avec l'énergie.

8. Implant (10) selon l'une des revendications 6 ou 7, **caractérisé en ce que** l'énergie nécessaire pour l'alimentation de l'unité de commande (30) est approvisionnée par l'intermédiaire de l'antenne (24) pour le transfert des signaux de commande et/ou du code d'activation ou l'antenne est mise dans le circuit pour le transfert de l'énergie de thérapie.

9. Implant (10) selon l'une des revendications précédentes, lequel dispose d'au moins un raccordement (34) pour le branchement d'un stimulateur cardiaque usuel.

10. Implant (10) selon l'une des revendications précédentes, lequel dispose d'un dispositif de mesure (32) qui est relié avec l'une des antennes (20, 34) et qui est conçu pour transmettre vers l'extérieur des données de mesure relatives au patient et/ou à l'appareil par l'intermédiaire de l'antenne (20, 24).

11. Générateur d'impulsions pour l'alimentation externe en énergie de thérapie d'un implant selon la revendication 1, comprenant
- un générateur haute fréquence pour la génération d'une tension alternative de haute fréquence, au moins
- une antenne, par laquelle l'énergie de thérapie est émise sous la forme d'une tension alternative haute fréquence en cas d'emploi, et
- une unité de commande, qui est conçue pour générer un signal de commande qui contient une information concernant la séquence de thérapie souhaitée,
parallèlement à un code d'activation, pour l'activation de l'implant.

12. Générateur d'impulsions selon la revendication 11, lequel est conçu pour pouvoir réceptionner et analyser, et/ou mettre en mémoire, des données de mesure, concernant le patient et/ou l'appareil, émises par un implant (10) selon la revendication 10.
